## Europäisches Patentamt

## European Patent Office

## Office européen des brevets

(19)

(11) Veröffentlichungsnummer: **0 345 247**
A2

(12) # EUROPÄISCHE PATENTANMELDUNG

(21) Anmeldenummer: **89890157.4**

(22) Anmeldetag: **01.06.89**

(51) Int. Cl.⁴: **A 61 K 31/095**
A 61 K 31/70, A 61 K 31/195

(30) Priorität: **03.06.88 AT 1453/88**

(43) Veröffentlichungstag der Anmeldung:
**06.12.89 Patentblatt 89/49**

(84) Benannte Vertragsstaaten:
**AT BE CH DE ES FR GB GR IT LI LU NL SE**

(71) Anmelder: **Birkmayer, Jörg, Univ.-Prof.DDr.**
**Schwarzspanierstrasse 15**
**A-1090 Wien (AT)**

(72) Erfinder: **Birkmayer, Jörg, Univ.-Prof.DDr.**
**Schwarzspanierstrasse 15**
**A-1090 Wien (AT)**

(74) Vertreter: **Haffner, Thomas M., Dr. et al**
**Patentanwaltskanzlei Dipl.-Ing. Adolf Kretschmer Dr.**
**Thomas M. Haffner Schottengasse 3a**
**A-1014 Wien (AT)**

(54) **Verwendung von monosubstituierten selenorganischen Verbindungen zur Herstellung eines Arzneimittels sowie daraus hergestelltes Arzneimittel und Verfahren zu seiner Herstellung.**

(57) Verwendung des Selenmethionins zur Herstellung eines zur Behandlung des Parkinson-Syndroms, der M. Alzheimer oder anderer Formen präseniler Demenz geeigneten Arzneimittels.

EP 0 345 247 A2

Bundesdruckerei Berlin

## Beschreibung

## Verwendung von monosubstituierten selenorganischen Verbindungen zur Herstellung eines Arzneimittels sowie daraus hergestelltes Arzneimittel und Verfahren zu seiner Herstellung

Die Erfindung bezieht sich auf die Verwendung von Selenmethionin, Selencystein oder anderen monosubstituierten selenorganischen Verbindungen, wie Selenaminosäuren, Selenproteine und Selenpolypeptide zur Herstellung eines zur Behandlung des Parkinson-Syndroms, des M. Alzheimer und anderer Formen der präsenilen Demenz geeigneten Arzneimittels. Weiters bezieht sich die Erfindung auf die gleichzeitige Verwendung der reduzierten Form des Nikotinamid-Adenindinucleotids (NADH) sowie auf ein Arzneimittel zur Behandlung des Parkinson-Syndroms, des M. Alzheimer und anderer Formen der präsenilen Demenz enthaltend Selenmethionin, Selencystein oder andere monosubstituierte selenorganische Verbindungen und gegebenenfalls NADH, und ein Verfahren zu seiner Herstellung.

Die Parkinson'sche Krankheit beruht auf einer gestörten dopaminergen Neurotransmission in den Basalganglien, im allgemeinen infolge eines zunehmenden Untergangs der dopaminergen Neuronen zunächst in der Substantia nigra, im Verlauf der Krankheit auch in anderen Arealen. In fortgeschrittenen Fällen wird auch ein zunehmender Noradrenalin-Mangel (z.B. im Locus caeruleus) feststellbar. Schon in frühen Stadien der Erkrankung findet man auch einen sehr deutlichen Abfall der Tyrosin-Hydroxylase-Aktivität, die interessanterweise auch in anderen Systemen und Organen (z.B. im Nebennierenmark) stark vermindert ist (siehe Tabelle 3 aus Birkmayer und Riederer, Die Parkinson-Krankheit, 2. Aufl., S. 34, Springer Verlag Wien, 1985).

Tabelle 3

*Tyrosinhyroxylase in verschiedenen Gebirnarealen beim Morbus Parkinson*

| Gehirnareale | | Kontrollen | M. Parkinson |
|---|---|---|---|
| N. caudatus | (15) | 27,8 ± 2,3 | 3,5 ± 1,0 (6)* |
| Putamen | (5) | 16,2 ± 5,9 | 1,2 ± 0,4 (6)* |
| S. nigra | (4) | 19,4 ± 6,2 | 4,9 ± 1,8 (4)* |
| L. caeruleus | (4) | 3,3 ± 0,1 | 2,0 ± 0,6 (2) |
| N. ruber | (5) | 5,7 ± 1,9 | 2,1 ± 1,4 (3) |
| Raphe + R.F. | (4) | 0,9 ± 0,6 | 1,5 ± 0,4 (5) |
| Hypothalamus | (5) | 3,1 ± 1,0 | 1,5 ± 0,3 (3) |
| C. mamillare | (5) | 0,6 ± 0,4 | 0,5 ± 0,9 (2) |
| N. accumbens | (5) | 2,0 ± 0,7 | 2,7 ± 2,2 (3) |
| Nebenniere (Medulla) | (5) | 186,2 ± 5,5 | 49,7 ± 12,4 (4)* |

Anzahl der Patienten in Klammern.

Mittelwerte ± sem (nmol Dopa/g-Gewebe.Stunde).

* $p < 0,01$.

Eine allgemein anerkannte Arbeitshypothese geht davon aus, daß die Basalganglien, insbesondere die Substantia nigra und andere Kerngebiete, durch Peroxyd-Radikale zerstört werden. Die Peroxyd-Radikale entstehen durch die Lipidperoxydation im Organismus, insbesondere im Hirn. Ein Enzym, das diese Peroxydation verhindert, ist die Glutathionperoxydase. Die Glutathionperoxydase ist ein selenhaltiges Enzym und kommt außerdem noch in den Erythrozyten vor. Der Selengehalt des Vollblutes gibt demnach Hinweis auf die Funktionsfähigkeit der Glutathionperoxydase im Erythrozyten und damit indirekt auch in den Basalganglien des Gehirns. Es wurde vor kurzem festgestellt, daß alle bisher untersuchten Parkinson-Patienten einen extremen Selenmangel im Vollblut aufweisen (1,1 bis 26 ng/l; Normbereich gesunde Erwachsene: 80 bis 120 ng/l).

Aus der DE-OS 35 42 309 ist die Anwendung eines medizinischen Antioxidativums bestehend aus einer Kombination von Selenmethionin und Verbindungen von selbstregenerierenden physiologischen Redoxsystemen zur Bekämpfung von freien Radikalen, welche für die Entstehung degenerativer Erkrankungen mitverantwortlich sind, bekanntgeworden.

Die US-PS 4 722 940 beschreibt die Herstellung von disubstituierten selenorganischen Verbindungen, wie Aminoalkylphenylseleniden, welche zur Behandlung von Funktionsstörungen des Nervensystems eingesetzt werden können.

Klinische Untersuchungen haben nun überraschenderweise ergeben, daß auch bei Patienten mit Parkinsonismus und schweren Schwankungen der Beweglichkeit durch die Gabe von Selenmethionin eine deutliche Besserung der Motorik erreicht wird.

Ein überraschender Synergismus zur Verabreichung von monosubstituierten selenorganischen Verbindungen hat sich bei gleichzeitiger Verabreichung von NADH ergeben. NADH wurde für sich genommen bereits zur Behandlung des Parkinsonismus vorgeschlagen, wobei jedoch die gleichzeitige Verabreichung von NADH und monosubstituierten selenorganischen Verbindungen eine deutlich bessere Wirkung zeigte.

Analoge Ergebnisse sind auch bei Patienten mit M. Alzheimer sowie anderen Formen präseniler Demenz erhoben worden. Alle diese Patienten weisen einen drastischen Mangel an Selen im Vollblut auf. 3 Tabletten täglich, enthaltend je 100 µg Selenmethionin, haben nach 4 Wochen zu einer eklatanten Verbesserung der intellektuellen Leistungsfähigkeit geführt. Gute Ergebnisse ließen sich mit Dosismengen von 5 bis 250 µg, vorzugsweise 25 bis 250 µg, je verabreichter Dosis erzielen, wobei bei gleichzeitiger Verwendung von NADH, dieses mit Vorteil in einem Verhältnis zu den verwendeten monosubstituierten selenorganischen Verbindungen zwischen 1:1 und 100:1 eingesetzt wurde.

Die klinischen Ergebnisse sind am Beispiel eines besonders eindrucksvollen Falles einer 58-jährigen Patientin mit M. Alzheimer (Fall 1) sowie einem Parkinson-Patienten (Fall 2) dargestellt.

**Fall 1: 58 Jahre**

Die Untersuchung fand am 10.04.1988 statt. Die Patientin war seit einigen Jahren vergeßlich und ihr Zustand verschlechterte sich. Die Patientin findet beim Einkaufen nicht mehr zurück, weiß auch nicht mehr was sie einkaufen muß und kann nicht mehr kochen. Sie findet sich in ihrer eigenen Wohnung nicht mehr zurecht und findet von einem Zimmer nicht in das andere und wünscht von ihrem Mann die Einlieferung in eine geschlossene Anstalt.

Objektiver Befund: Lesen nicht möglich, erkennt nicht einmal Buchstaben.

Rechnen: 4 x 8 = 48, 10 - 2 = 2, Sprichwort-Deutung: Der Apfel fällt nicht weit von Stamm - kann nicht einmal den Satz wiederholen. Sie kann weder den Wochentag noch den Monat angeben. Fernsehen und Radio keine Aufnahmemöglichkeit. Blutwert für Selen 6,6 ng, organisch neurologisch o.B.
Diagnose M. Alzheimer
Therapie: Selen 3 Tabletten täglich. à 100 ng Selenmethionin,
Kontrolle 16.05.1988:
Stimmung ausgeglichen - euphorisch, kann wieder kochen und einkaufen. 4 + 8 = 12. Wochentage werden richtig angegeben. Nachrichten im Fernsehen versteht sie wieder. Sprachlicher Ausdruck wesentlich vielfältiger, keine Wortarmut.
Die Therapie mit selenorganischen Verbindungen wird fortgesetzt.

**Fall 2: 62 Jahre**

Seit 12 Jahren Parkinson-Krankheit. Untersuchung am 14.04.1988. Disability 75, Therapie Madopar (R) 125 (=L-DOPA + Benserazid) 3-mal, Oxyferriscorbone (siehe Beipackzettel) eine Ampulle i.m. täglich, 3 Tabletten Selenmethionin täglich (à 100 ng).
Kontrolle 08.05.1988, Disability 40. Gehen kaum behindert. Stoßen nach oben ohne Behinderung, Springen normal, Sprache Startschwierigkeiten, aber dann normal. Haltung, Mimik, Starten normal. Erster Selenwert im Blut am 14.04.1988 1,1 ng, Kontolle am 08.05.1988 18,6 ng.

Die Daten der Patienten zeigen, daß die Einnahme von Selenmethionin zu einer deutlichen Verbesserung der Disability (Bewegungsunfähigkeit) führt. Dies wird sowohl in der on- wie auch in der off-Phase festgestellt und wird sowohl bei gleichzeitiger Gabe von Standard-Präparaten wie auch ohne gleichzeitige Gabe von bekannten Parkinsonmitteln erreicht. Unter der erfindungsgemäßen Medikation wird auch eine Verkürzung der off-Phase erreicht.

Zur erfindungsgemäßen Verwendung kann das Selenmethionin oder seine physiologischen Analoga in üblicher Weise mit pharmazeutisch annehmbaren Hilfs- und Trägerstoffen konfektioniert werden. Gegebenenfalls kann Selenmethionin auch in Kombination mit anderen Wirkstoffen, wie NADH oder NADPH oder postsynaptischen Dopaminagonisten, wie Lisurid oder Amorphin, verwendet werden.

Besonders bei der Verwendung von NADH aber auch bei der Verwendung von NADPH in Kombination mit monosubstituierten selenorganischen Verbindungen wurden drastische Verbesserungen und damit eine synergistische Wirkung beobachtet.

Zur Verwendung als Arzneimittel kann Selenmethionin in üblichen galenischen Zubereitungsformen für die orale, parenterale (wie intravenös oder subkutan) oder sublinguale Anwendung eingearbeitet werden. Die Präparate können in fester Form als Tabletten, Kapseln, Dragées oder in flüssiger Form als Lösungen, Suspensionen, Sprays oder Emulsionen sowie Zubereitungsformen mit verzögerter Wirkstoffgabe vorliegen. Zur Verbesserung der Disability bei Parkinson-Patienten und der mentalen Leistungsfähigkeit bei Alzheimer-Patienten oder solchen mit anderen Formen präseniler Demenz beträgt die Einzeldosis bei oraler Applikation zwischen 5 und 250 µg, vorzugsweise zwischen 25 und 250 µg und die Tagesdosis zwischen 5 und 500 µg, vorzugsweise 25 und 300 µg.

Liegt das Selen in Form von Selenmethionin oder Selenserin oder einer anderen biologisch vorkommenden Organoverbindung vor, so sind hiefür alle bekannte saure und basische Salzbildner geeignet, wie organische und anorganische Säuren, wie z.B.. Seleneneicosanosäure, Selenheptadecansäure, Selenlysin, Selenprolin, Selenbiotin, Selencystamin, Selencystein, Selencystin, Selendiglutathion, Selenenzym, Selenfolat, Selenethylen, Selenglycoprotein, Selenguanosin, Selenhomocystein, Selenmethylcholesterol, Selennucleosid, Selenthyrosin.

Bei der galenischen Zubereitung scheint es mit Rücksicht auf den Wirkungsmechanismus monosubstituierter selenorganischer Verbindungen besonders vorteilhaft, möglichst hydrophobe bzw. lipophile Trägerstoffe zu wählen. Besonders bevorzugt erscheinen in diesem Zusammenhang Lecithine, Phospholipide, Cholin, Acetylcholin. Ein Beispiel für eine geeignete galenische Zubereitung wird nachfolgend angegeben.

Die für orale Verabreichung geeigneten Tabletten, welche die nachfolgend beschriebenen Bestandteile enthalten, werden in an sich bekannter Weise hergestellt.

| Bestandteile | Gewicht |
| --- | --- |
| Selenmethionin | 0,25 mg |
| Laktose | 98,05 mg |
| Maisstärke | 14,00 mg |
| Polyvinylpyrrolidon | 5,00 mg |
| Magnesiumstearat | 0,70 mg |
| Talk | 2,00 mg |
|  | 120,00 mg |

**Patentansprüche**

1. Verwendung von Selenmethionin, Selencystin oder anderen monosubstituierten selenorganischen Verbindungen, wie Selenaminosäuren, Selenproteine und Selenpolypeptide zur Herstellung eines zur Behandlung des Parkinson-Syndroms, des M. Alzheimer und anderer Formen der präsenilen Demenz geeigneten Arzneimittels.

2. Verwendung nach Anspruch 1, dadurch gekennzeichnet, daß zusätzlich zu den monosubstituierten selenorganischen Verbindungen NADH eingesetzt wird.

3. Verwendung nach Anspruch 2, dadurch gekennzeichnet, daß das Verhältnis NADH zu monosubstituierten selenorganischen Verbindungen zwischen 1:1 und 100:1 gewählt wird.

4. Arzneimittel zur Behandlung des Parkinson-Syndroms, des M. Alzheimer und anderer Formen der präsenilen Demenz enthaltend Selenmethionin, Selencystein oder andere monosubstituierte selenorganische Verbindungen und gegebenenfalls NADH.

5. Arzneimittel nach Anspruch 4, dadurch gekennzeichnet, daß die monosubstituierten selenorganischen Verbindungen in einer Menge von 5 bis 250 μg, vorzugsweise 25 bis 250 μg, je Dosiseinheit enthalten sind.

6. Verfahren zur Herstellung eines Arzneimittels gemäß Anspruch 3, 4 oder 5, dadurch gekennzeichnet, daß man Selenmethionin, Selencystein oder andere monosubstituierte selenorganische Verbindungen, wie Selenaminosäuren, Selenproteine und Selenpolypeptide zur Herstellung eines zur Behandlung des Parkinson-Syndroms, des M. Alzheimer und anderer Formen der präsenilen Demenz geeigneten Arzneimittels mit geeigneten Träger-, Hilfs- und/oder Zusatzstoffen in eine geeignete Darreichungsform bringt.